# EUROPÄISCHE PATENTSCHRIFT

(11) **EP 2 464 287 B1**
(45) Veröffentlichungstag und Bekanntmachung des Hinweises auf die Patenterteilung: **03.01.2018**
(21) Anmeldenummer: 10747160.9
(22) Anmeldetag: 05.08.2010
(51) Int. Cl.: A61B 5/083, A61B 5/097

(54) **ANWENDEREINHEIT ZUR BESTIMMUNG VON LEISTUNGSPARAMETERN AUS ATEMGASANALYSEN**
USER UNIT FOR DETERMINING OUTPUT PARAMETERS FROM BREATH GAS ANALYSES
UNITÉ UTILISATEUR POUR LA DÉTERMINATION DES PARAMÈTRES DE PERFORMANCE DANS LES ANALYSES DES GAZ DE RESPIRATION

(30) Priorität: 11.08.2009 DE 102009036901; 07.10.2009 DE 102009048603; 13.01.2010 DE 102010004611
(43) Veröffentlichungstag der Anmeldung: 20.06.2012
(73) Patentinhaber: aeroscan GmbH, 10785 Berlin (DE)
(72) Erfinder: VÖLKEL, Michael, Haltern am See - Hullern (DE); TUCHTENHAGEN, Dirk, 90762 Fürth (DE)
(74) Vertreter: Von Rohr Patentanwälte Partnerschaft mbB
(86) Internationale Anmeldenummer: PCT/EP2010/004803
(87) Internationale Veröffentlichungsnummer: WO 2011/018186

(56) Entgegenhaltungen:
- GB-A- 2 291 807
- US-A1- 2003 183 227
- US-A1- 2008 149 105
- US-B1- 6 302 103

## Beschreibung

Die Erfindung betrifft eine Anwendereinheit zur Bestimmung von Leistungsparametern aus Atemgasanalysen, insbesondere für den Einsatz in Spiroergometriegeräten, mit einem vorzugsweise auswechselbaren Atemrohr und einem Gehäuseteil, wobei, weiter vorzugsweise, das Atemrohr in das Gehäuseteil einführbar ist, um die Anwendereinheit in einen Meßzustand zu überführen.

Ein genaues und direktes Verfahren zur Bestimmung der Stoffwechselaktivität des Menschen ist die Analyse der Atemgase, bei der die Konzentrationen von Sauerstoff und Kohlenstoffdioxid im Atem sowie der Volumenstrom der Atmung bestimmt werden. Aus den Messungen können Stoffwechselkennwerte wie beispielsweise der respiratorische Quotient, auch als RQ bekannt, errechnet werden. Der RQ ist das Verhältnis der abgeatmeten Kohlenstoffdioxidmenge zur aufgenommenen Sauerstoffmenge. Zur Bestimmung dieser Gasmengen werden verschiedene Parameter gemessen. Mit einem Sensor, der beispielsweise über Ultraschallaufzeitmessung die Strömungsgeschwindigkeit des Atemgases mißt, wird der Volumenstrom des Atemgases bestimmt. Durch Integration des Volumenstroms über die Zeit können verschiedene Atemvolumina abgeleitet werden. In bekannten Spiroergometriegeräten wird zudem aus dem Hauptatemstrom eine Gasprobe abgesaugt und der im Gerät enthaltenen Sensorik zugeführt. Dadurch können die Konzentrationen von Sauerstoff und Kohlenstoffdioxid bei Ein- und Ausatmung bestimmt werden. Die jeweiligen Werte der Gaskonzentrationen unterscheiden sich wesentlich zwischen Ein- und Ausatmung. Über das zuvor bestimmte Atemvolumen können aus den Konzentrationen die Gasmengen berechnet werden, die vom Körper umgesetzt wurden.

Herkömmliche Spiroergometriegeräte bestehen aus einer Anwendereinheit und einer Analyseeinheit, wobei die Analyseeinheit die für die Bestimmung der Gaskonzentrationen notwendige Sensorik enthält. Die Probandenschnittstelle ist meistens eine Anwendereinheit mit Atemmaske, an der zusätzlich ein Flowsensor angebracht ist, der die Atemströmung mißt. Mittels eines dünnen Schlauches an der Atemmaske wird kontinuierlich eine Gasprobe abgesaugt und der Analyseeinheit zugeführt, in der die entsprechenden Gaskonzentrationen gemessen werden. Anwendereinheiten mit Atemmaske werden insbesondere bei kontinuierlichen Messungen über einen längeren Zeitraum eingesetzt.

Zur Bestimmung von Leistungsparametern aus Atemgasanalysen ist es erforderlich, den gesamten Atemstrom zu vermessen. Im Normalfall wird der Atemstrom beim Ausatmen auf einen Teil Nasenatmung und einen Teil Mundatmung aufgeteilt, wobei zur Bestimmung der Leistungsparameter beide Ströme erfaßt werden müssen. Hierzu decken bekannte Atemmasken sowohl den Mund als auch die Nase eines Probanden ab. Damit wird der gesamte Atemstrom durch die Maske geleitet. Um Undichtigkeiten zu vermeiden, muß die Maske eng am Gesicht des Probanden anliegen. Eine sichere Befestigung am Kopf des Probanden wird durch elastische Bänder erreicht. Die eng anliegende Atemmaske kann jedoch für den Probanden ausgesprochen unangenehm sein. Zudem hat sie hygienische Nachteile, da sie nach jeder Anwendung durch den Atem der Versuchsperson kontaminiert ist. Sollen mehrere Personen nacheinander getestet werden, so muß die Atemmaske nach jeder Benutzung desinfiziert werden, was aufwendig ist. Zudem kann es durch unzureichende Desinfektion der Atemmaske zu einer Infektion kommen.

Für sogenannte Spotmessungen, bei denen jeweils nur zu den Meßzeitpunkten die Leistungsparameter aus Atemgasen analysiert werden, können Nasenklemmen eingesetzt werden, um den normalerweise über die Nase abströmenden Atemstrom zu unterdrücken. Nasenklemmen können auch zusammen mit solchen Anwendereinheiten eingesetzt werden, die ein vorzugsweise auswechselbares Atemrohr und ein Gehäuseteil aufweisen, wobei das Atemrohr in das Gehäuseteil eingesetzt ist. Weiter vorzugsweise kann dabei das Atemrohr für den Einmalgebrauch vorgesehen sein. Eine derartige Anwendereinheit ist beispielsweise aus der DE 42 22 286 C1 bekannt.

Aus der WO 2009/056562 A1 ist eine Nasenklemme für den Einmalgebrauch bekannt, die aus einfachen Kunststoffplatten durch herkömmliche Verfahren wie Fräsen, Schneiden oder Stanzen gefertigt werden kann. Die bekannte Nasenklemme weist eine im wesentlichen hufeisen- oder U-förmige Form auf. Von einer Basis erstrecken sich zwei Kragarme in eine im wesentlichen gleiche Richtung. Zwischen diesen beiden Kragarmen der Nasenklemme befindet sich ein offener Bereich, der zum Einführen der Nase ausgebildet ist.

Aus der DE 34 16 146 A1 ist eine weitere Nasenklemme bekannt, die zwei durch eine Feder zusammengehaltene Schenkel aufweist, wobei die Schenkel aus einem leichten Material gefertigt und die Feder gerade so stark bemessen ist, daß sie die Nasenwände zum Verschließen der Nasenöffnungen zusammendrücken kann. Durch eine Einstellschraube kann der Druck der Feder individuell verändert werden.

Die bekannten Nasenklemmen zeichnen sich gegenüber Atemmasken durch einen höheren Tragekomfort aus. Die Verwendung der bekannten Nasenklemmen zur Einmalbenutzung ermöglicht die Bestimmung von Leistungsparametern aus Atemgasanalysen unter Sicherstellung der notwendigen Hygiene. Allerdings kann es beim Aufsetzen der bekannten Nasenklemmen auf die Nase zu Anwenderfehlern kommen, die dazu führen können, daß die Nase nicht vollständig verschlossen ist. Die Nasenatmung ist dann auch bei aufgesetzter Nasenklemme nicht vollständig ausgeschlossen, so daß es zu einer Verfälschung der Meßergebnisse durch einen über die Nase fließenden Luftstrom bzw. Atemstrom kommen kann.

Eine weitere Anwendereinheit ist aus GB-A-2291807 bekannt. Diese Anwendereinheit umfasst ein auswechselbares Atemrohr und ein Gehäuseteil, sowie eine Kinnstütze und auch eine Nasenklemme.

Aufgabe der vorliegenden Erfindung ist es, eine Anwendereinheit der eingangs genannten Art zur Verfügung zu stellen, die von einem Probanden in einfacher Weise zu handhaben ist und sich bei der Bestimmung der Leistungsparameter durch einen hohen Tragekomfort und durch eine hohe Meßgenauigkeit auszeichnet. Im übrigen soll die erfindungsgemäße Anwendereinheit kostengünstig herstellbar sein und hohen Anforderungen an die Hygiene genügen.

Die vorgenannten Aufgaben sind bei einer Anwendereinheit der eingangs genannten Art bei einer ersten Ausführungsform der Erfindung dadurch gelöst, daß auf der Oberseite des Gehäuseteils eine mit dem Gehäuseteil verbundene Nasenklemme ausgebildet zum Verschließen der Nase bei der Bestimmung der Leistungsparameter vorgesehen ist. Der Erfindung liegt der Grundgedanke zugrunde, die Nasenklemme mit dem Gehäuseteil der Anwendereinheit zu verbinden, so daß Anwenderfehler beim Aufsetzen der Nasenklemme auf die Nase weitgehend ausgeschlossen werden können. Das Anlegen der Nasenklammer und das Einführen des Mundstücks kann mit einer Handbewegung erfolgen, was insbesondere unter körperlicher Belastung z.B. auf einem Laufband eine große Erleichterung für den Probanden darstellt. Durch die Haltung der Anwendereinheit bei der Bestimmung der Leistungsparameter, d. h. die Anordnung des Gehäuseteils und des Atemrohrs relativ zu dem Probanden, wird auch die relative Lage der Nasenklemme zu dem Probanden festgelegt. Die erfindungsgemäße Anwendereinheit ist angenehm zu handhaben und ermöglicht eine Bestimmung der Leistungsparameter mit hoher Genauigkeit, wobei eine Verfälschung der Meßergebnisse durch einen über die Nase fließenden Atemstrom weitgehend ausgeschlossen werden können. Zudem können die mit der Nase des Probanden in Kontakt kommenden Teile der Nasenklemme für den Einmalgebrauch ausgelegt sein, um hohen Anforderungen an die Hygiene zu genügen.

Die erfindungsgemäße Anwendereinheit weist ein Atemrohr auf, das vorzugsweise für den Einmalgebrauch vorgesehen ist, und ein Gehäuseteil, in das das Atemrohr eingesetzt bzw. eingeschoben werden kann. An dem Atemrohr und/oder an dem Gehäuseteil kann wenigstens eine Entnahmestelle zur Entnahme einer Gasprobe aus dem Hauptatemstrom vorgesehen sein. Die entnommene Gasprobe kann dann über eine Schlauchleitung an eine separate Analyseeinheit weitergeleitet werden. In der Analyseeinheit kann dann eine Analyse der Atemgase erfolgen. Grundsätzlich ist es aber auch möglich, daß die Anwendereinheit Sensoren zur Analyse der Atemgase aufweist. Im übrigen können an dem Gehäuseteil Sensoren, insbesondere Ultraschallwandler, vorgesehen sein, mit denen es möglich ist, die Geschwindigkeit der Atemströmung, den Volumenstrom und verschiedene Lungenvolumina zu berechnen. Die Gaskonzentrationen in der Atemgasprobe, die Geschwindigkeit der Atemströmung, der Atemvolumenstrom und verschiedene Lungenvolumina können zu den Leistungsparametern zählen, die unter Verwendung der erfindungsgemäßen Anwendereinheit bestimmt werden sollen, wobei die Aufzählung nicht abschließend ist.

Zur Lösung der oben genannten Aufgaben ist bei einer Anwendereinheit der eingangs genannten Art bei einer zweiten alternativen Ausführungsform der Erfindung vorgesehen, daß auf der Unterseite des Gehäuseteils eine mit dem Gehäuseteil verbundene Kinnstütze ausgebildet zur Anlage gegen das Kinn eines Benutzers bei der Bestimmung der Leistungsparameter vorgesehen ist. Durch die Kinnstütze wird die Anwendereinheit bei der Bestimmung der Leistungsparameter gegen das Kinn des Probanden abgestützt, so daß ein geringer Kraftaufwand zum Halten der Anwendereinheit erforderlich ist. Dies trägt zu einer einfachen Handhabung der erfindungsgemäßen Anwendereinheit bei. Im übrigen kann durch eine geeignete Formgebung der Kinnstütze eine gewünschte Stellung der Anwendereinheit relativ zu dem Gesicht des Probanden unterstützt werden. Dies ist insbesondere dann von Vorteil, wenn das Gehäuse auf der Oberseite eine mit dem Gehäuseteil verbundene Nasenklemme aufweist, so daß das Gehäuseteil nach dem Einführen des Atemrohrs in den Mund des Probanden über die Nasenklemme an der Nase des Probanden gehalten und über die Kinnstütze am Kinn des Probanden abgestützt ist. Die Kinnstütze kann dazu beitragen, daß die Anwendereinheit mit der Nasenklemme exakt zum Gesicht bzw. zur Nase des Probanden ausgerichtet ist und die Nase des Probanden stets vollständig durch die Nasenklemme verschlossen wird. Dadurch werden die Nasenatmung ausgeschlossen und eine hohe Genauigkeit bei der Bestimmung der Leistungsparameter sichergestellt.

Wenngleich die Nasenklemme und die Kinnstütze vorzugsweise an dem Gehäuseteil befestigt sind, wobei das Gehäuseteil für eine mehrfache Benutzung ausgebildet und vorgesehen ist, ist es grundsätzlich möglich, daß die Nasenklemme und/oder die Kinnstütze an dem Atemrohr befestigt und auch für einen Einmalgebrauch vorgesehen sein können. Diesem Aspekt kommt eigenerfinderische Bedeutung zu.

Die vorgenannten Aspekte und Merkmale der vorliegenden Erfindung sowie die nachfolgend beschriebenen Aspekte und Merkmale der vorliegenden Erfindung können unabhängig voneinander, aber auch in einer beliebigen Kombination realisiert werden. Weitere Vorteile, Merkmale, Eigenschaften und Aspekte der vorliegenden Erfindung ergeben sich aus der folgenden Beschreibung einer bevorzugten Ausführungsform anhand der Zeichnung. Es zeigen:
- Fig. 1: eine perspektivische Darstellung einer erfindungsgemäßen Anwendereinheit in einer Ansicht schräg von oben,
- Fig. 2: eine schematische Darstellung der in Fig. 1 dargestellten Anwendereinheit bei der Bestimmung von Leistungsparametern eines Probanden,
- Fig. 3: eine perspektivische Darstellung einer Nasenklemme der in Fig. 1 dargestellten Anwendereinheit mit nach oben verschobenen Klemmrollen,
- Fig. 4: die in Fig. 3 dargestellte Nasenklemme mit nach unten verschobenen Klemmrollen,
- Fig. 5: eine Teilansicht eines Klemmbügels der in den Fig. 3 und 4 dargestellten Nasenklemme mit zwei Rollkörpern in einem teilmontierten Zustand der Rollkörper,
- Fig. 6: eine Teilansicht eines Rollkörpers aus Fig. 5 im montierten Zustand,
- Fig. 7: eine Teilansicht des in Fig. 5 dargestellten Klemmbügels im montierten Zustand der Rollkörper,
- Fig. 8: eine Teilansicht eines Klemmschenkels einer in den Fig. 3 und 4 dargestellten Nasenklemme mit einem anderen Rollkörper in einem demontierten Zustand des Rollkörpers und
- Fig. 9: den in Fig. 8 dargestellten Klemmschenkel mit dem Rollkörper in einem montierten Zustand des Rollkörpers.

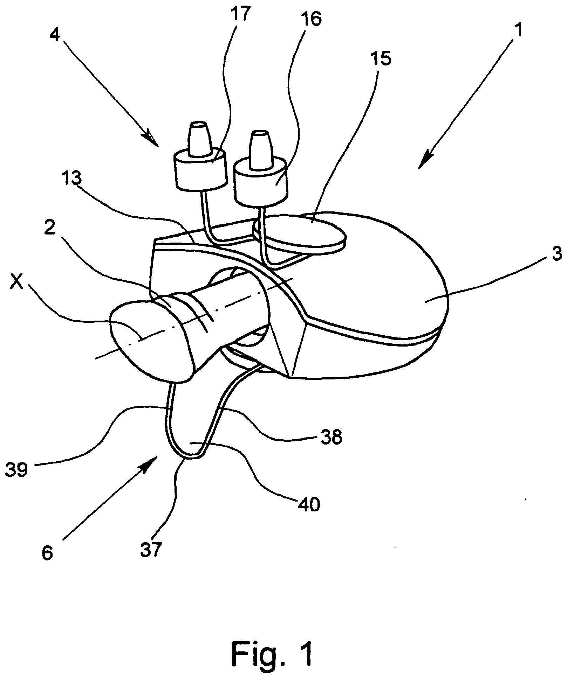

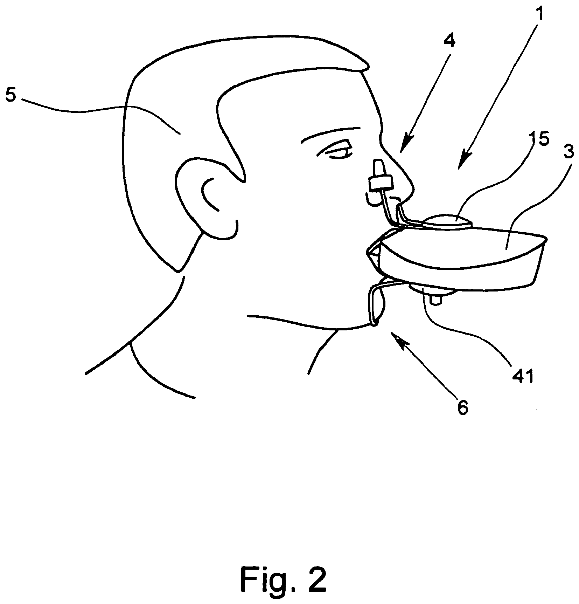

In den Fig. 1 und 2 ist schematisch eine Anwendereinheit 1 zur Verwendung bei der Bestimmung von Leistungsparametern aus Atemgasanalysen, insbesondere für den Einsatz in Spiroergometriegeräten, gezeigt. Die Anwendereinheit 1 weist ein vorzugsweise auswechselbares Atemrohr 2 und ein Gehäuseteil 3 auf, wobei das Atemrohr 2 in das Gehäuseteil 3 einführbar bzw. einschiebbar ist, um die Anwendereinheit 1 in einen Gebrauchs- bzw. Meßzustand zu überführen.

Auf der Oberseite des Gehäuseteils 3 ist eine mit dem Gehäuseteil 3 verbundene Nasenklemme 4 vorgesehen. Die Nasenklemme 4 dient zum Verschließen der Nase eines Benutzers 5 bei der Bestimmung der Leistungsparameter. Wie sich weiter aus den Fig. 1 und 2 ergibt, kann auf der Unterseite des Gehäuseteils 3 eine mit dem Gehäuseteil 3 verbundene Kinnstütze 6 ausgebildet zur Anlage gegen das Kinn des Benutzers 5 bei der Bestimmung der Leistungsparameter vorgesehen sein. Die in den Fig. 1 und 2 dargestellte Anwendereinheit 1 läßt sich in einfacher Weise von einem Benutzer 5 während der Bestimmung der Leistungsparameter handhaben. Bei der Bestimmung der Leistungsparameter ist es insbesondere nicht erforderlich, daß der Benutzer das Gehäuseteil 3 mit den Händen festhält. Das Gehäuseteil 3 wird über das Atemrohr 2 mit dem Mund und zusätzlich über die Nasenklemme 4 an der Nase gehalten und ist dabei über die Kinnstütze 6 am Kinn des Benutzers 5 abgestützt. Durch die Nasenklemme 4 wird dabei die Nasenatmung bei der Bestimmung der Leistungsparameter ausgeschlossen. Dadurch trägt die Anwendereinheit 1 zu einer hohen Genauigkeit bei der Bestimmung der Leistungsparameter bei.

Nicht dargestellt ist im übrigen, daß die Anwendereinheit 1 über eine Schlauchverbindung mit einer Analyseeinheit verbunden sein kann, die eine Absaugeinrichtung und die für die Bestimmung von Gaskonzentrationen im Atemgas notwendige Sensorik enthalten kann. Im übrigen können in dem Gehäuseteil 3 Meßeinrichtungen, wie beispielsweise Ultraschallwandler, vorgesehen sein, um in an sich aus dem Stand der Technik bekannter Weise den Volumenstrom und/oder Lungenvolumina beim Einatmen und/oder beim Ausatmen zu bestimmen.

Nicht dargestellt ist im übrigen, daß die Anwendereinheit 1 bei einer alternativen Ausführungsform auch lediglich eine mit dem Gehäuseteil 3 verbundene Nasenklemme 4 oder eine mit dem Gehäuseteil 3 verbundene Kinnstütze 6 aufweisen kann.

Die Nasenklemme 4 kann in Längsrichtung X₁ des Atemrohres 2 tiefenverstellbar an dem Gehäuseteil 3 gelagert und an unterschiedlichen Stellen am Gehäuseteil 3 festsetzbar sein. Dadurch läßt sich der Abstand der Nasenklemme 4 vom Gesicht des Benutzers 5 verändern und die Position der Nasenklammer 4 relativ zur Position der Nase des Benutzers 5 vorgeben. Im übrigen kann es vorgesehen sein, daß die Nasenklemme 4 zusätzlich zur Tiefenverstellbarkeit auch quer zur Längsrichtung X₁ des Atemrohres 2 bzw. in radialer Richtung verstellt werden kann, um eine Anpassung an die Nasenform zu erreichen. Dadurch kann ein vollständiger Verschluß der Nase bei der Messung der Leistungsparameter erreicht werden.

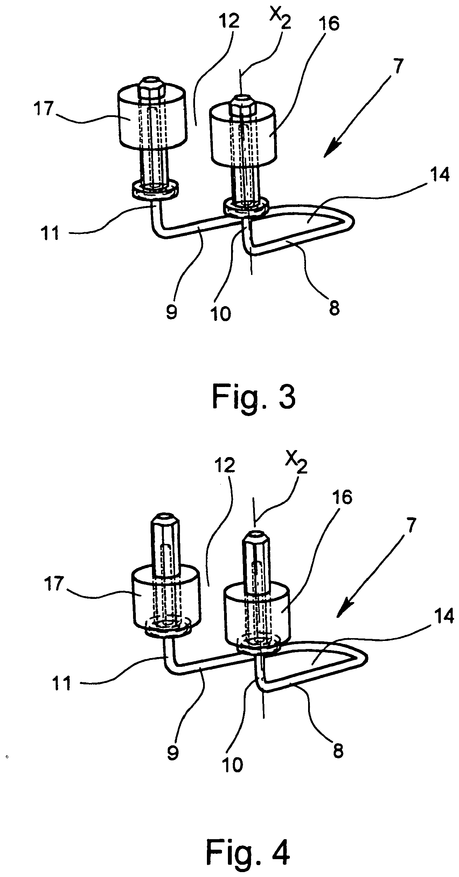

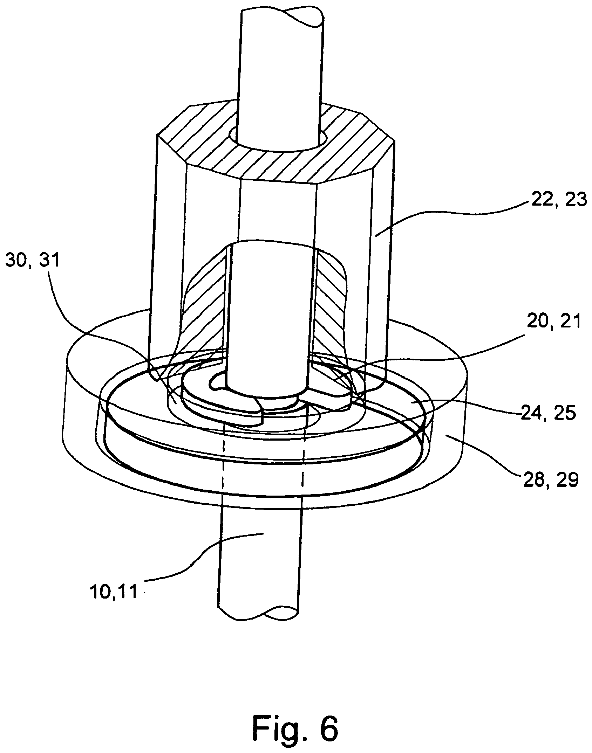

Wie sich aus den Fig. 3 bis 9 ergibt, kann die Nasenklemme 4 einen im wesentlichen U-förmigen Klemmbügel 7 mit zwei Klemmschenkeln 8, 9 aufweisen, wobei die beiden Klemmschenkel 8, 9 an den freien Enden 10, 11 L-förmig abgewinkelt sein können. Zwischen den abgewinkelten Enden 10, 11 wird dann ein zur Seite eines Mundstücks des Atemrohrs 2 bzw. zur Seite des Benutzers 5 offener Klemmbereich 12 zum Einführen der Nase des Benutzers 5 gebildet. Als Klemmbügel 7 kann ein gebogener Stahldraht, vorzugsweise ein Edelstahldraht, vorgesehen sein mit einem Durchmesser von 1 mm bis 5 mm, vorzugsweise von ca. 2 mm. Dadurch wird eine ausreichend hohe Klemmkraft der Klemmschenkel 8, 9 gewährleistet.

Wie sich aus den Fig. 1 und 2 ergibt, können die freien Enden 10, 11 der Klemmschenkel 8, 9 unter einem Winkel von kleiner oder gleich 90° zur Mittellängsachse X₁ des Atemrohrs 2 angeordnet sein. Die freien Enden 10, 11 der Klemmschenkel 8, 9 erstrecken sich dann nach vorne über einen stirnseitigen Außenrand 13 des Gehäuseteils 3 hinweg, so daß das Einführen der Nase des Benutzers 5 in den Klemmbereich 12 in einfacher Weise möglich ist. Das Gehäuseteil 3 kann auf der dem Benutzer 5 zugewandten Stirnseite zusätzlich eine konkave Wölbung aufweisen, die es ermöglicht, das Gesicht des Benutzers 5 näher an die Nasenklemme 4 heranzuführen.

Um eine einfache Tiefenverstellung der Nasenklemme 4 zu ermöglichen, kann der Klemmbügel 7 über eine Verschraubung mit wenigstens einer Klemmschraube an dem Gehäuseteil 3 festgesetzt sein, vorzugsweise in einem U-förmigen Verbindungsbereich 14 der beiden Klemmschenkel 8, 9. Der U-förmige Verbindungsbereich 14 ist in den Fig. 3 und 4 gezeigt. Die Klemmschraube wird im Bereich zwischen den beiden Klemmschenkeln 8, 9 in das Gehäuseteil 3 eingeschraubt, wobei der Klemmbügel 7 zwischen einem Schraubenkopf 15 der Klemmschraube und dem Gehäuseteil 3 eingespannt wird. Dabei läßt sich der Klemmbügel 7 in Längsrichtung des Atemrohrs 2 vor dem Festziehen der Klemmschraube mehr oder weniger weit nach vorne oder nach hinten verschieben, jeweils mit Bezug auf das Gesicht des Benutzers 5. Durch das Einspannen des Klemmbügels 7 zwischen dem Schraubenkopf 15 und dem Gehäuseteil 3 wird der Klemmbügel 7 unverlierbar am Gehäuseteil 3 gehalten und durch Festziehen der Klemmschraube mit einem gewünschten Abstand zur Nase des Benutzers 5 fixiert. Durch einen optimalen Abstand der freien Enden 10, 11 der Nasenklemme 4 von der Nase des Benutzers 5 wird ein bequemer Sitz der Nasenklemme 4 ermöglicht, so daß die Klemmwirkung durch den Benutzer 5 als nicht-störend empfunden wird. Im übrigen wird durch einen optimalen Abstand der Nasenklemme 4 zu dem Gesicht des Benutzers 5 ein möglichst vollständiger Verschluß der Nase bei der Bestimmung der Leistungsparameter gewährleistet.

Nicht im einzelnen dargestellt ist, daß an dem Gehäuseteil 3 und/oder an dem Schraubenkopf 15 wenigstens ein Führungsvorsprung und/oder eine Führungsausnehmung zur Führung der Klemmschenkel 8, 9 vorgesehen sein kann. Dadurch kann eine stets mittige Anordnung des Klemmbügels 7 relativ zum Gehäuseteil 3 und zum Atemrohr 2 bei der Tiefeneinstellung sichergestellt werden. Im übrigen läßt sich über eine Klemmung an dem Gehäuseteil 3 durch entsprechende Führungsvorsprünge und/oder Führungsausnehmungen auch eine bestimmte Aufspreizung der Klemmschenkel 8, 9 beim Verschieben in Längsrichtung des Atemrohrs 2 erzwingen.

Wie sich insbesondere aus den Fig. 3 und 4 ergibt, kann jeder Klemmschenkel 8, 9 an seinem abgewinkelten Ende 10, 11 eine um die Längsachse des Klemmschenkels 8, 9 drehbare Klemmrolle 16, 17 aufweisen, wobei zwischen den Klemmrollen 16, 17 der Klemmbereich 12 gebildet wird. Der Abstand zwischen den Klemmrollen 16, 17 ist dabei so bemessen, daß einerseits die Nase zuverlässig verschlossen wird, aber andererseits die Klemmwirkung durch den Probanden nicht als unangenehm empfunden wird. Das Aufschieben der Nasenklemme 4 auf die Nase wird dabei durch die drehbare Lagerung der Klemmrollen 16, 17 vereinfacht, wobei die Umfangsflächen der Klemmrollen 16, 17 auf den Nasenaußenwänden abrollen.

Um einen möglichst vollständigen Verschluß der Nase zu gewährleisten, können die Klemmrollen 16, 17 zylinderförmig ausgebildet sein. Zu dem gleichen Zweck kann vorzugsweise vorgesehen sein, daß die Klemmrollen 16, 17 als elastischer Formkörper, insbesondere aus Schaumstoff, hergestellt sind. Um die Position der Klemmrollen 16, 17 dem Gesicht und der Nase anzupassen, können diese in Richtung der Längsachse X₂ der Klemmschenkel 8, 9 verschiebbar gelagert sein. Dies ergibt sich aus einem Vergleich der Fig. 3 und 4.

Für eine drehbare Lagerung der Klemmrollen 16, 17 können an den abgewinkelten Enden 10, 11 der Klemmschenkel 8, 9 und um die Längsachse X₂ der Klemmschenkel 8, 9 drehbar gelagerte zylindrische Rollkörper 18, 19 vorgesehen sein, wobei jede Klemmrolle 16, 17 eine mittlere Öffnung zum Aufstecken auf den Rollkörper 18, 19 aufweist. Für eine Anpassung der Position der Klemmrollen 16, 17 an das Gesicht und die Nase des Benutzers 5 können die Klemmrollen 16, 17 in axialer Richtung verschiebbar an den Rollkörpern 18, 19 gehalten sein. Zu diesem Zweck weist der Rollkörper 18, 19 eine größere Länge auf als die Klemmrolle 16, 17. Zudem ist es möglich, die Klemmrollen 16, 17 für den Einmalgebrauch einzusetzen, wobei die Klemmrollen 16, 17 nach einem Gebrauch von den Rollkörpern 18, 19 abgezogen und gegen ungebrauchte Klemmrollen 16, 17 ausgetauscht werden können. Dadurch wird eine hohe Hygiene bei der Bestimmung der Leistungsparameter sichergestellt.

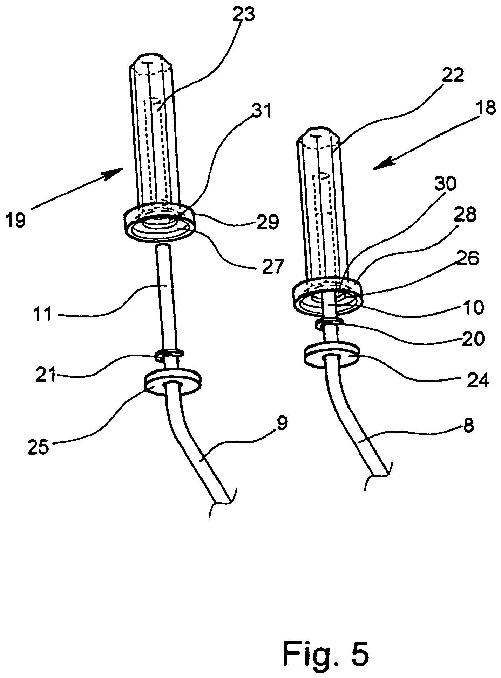

In den Fig. 5 bis 7 sind Rollkörper 18, 19 einer ersten Ausführungsform dargestellt. Die Rollkörper 18, 19 werden durch ein Sicherungselement 20, 21 gegen axiales Verschieben bzw. Abrutschen auf dem Klemmschenkel 8, 9 gesichert. Bei der dargestellten Ausführungsform ist als Sicherungselement 20, 21 ein in einer Nut des Klemmschenkels 8, 9 festgelegter Wellensicherungsring vorgesehen.

Der Rollkörper kann zweiteilig ausgebildet sein und einen zylinderförmigen Lagerabschnitt 22, 23 für die Klemmrollen 16, 17 und ein ringförmiges Verschlußteil 24, 25 aufweisen. Bei dem Verschlußteil 24, 25 kann es sich um eine Kunststoffunterlegscheibe handeln.

Zur Montage der Rollkörper 18, 19 wird zunächst das Verschlußteil 24, 25 auf das abgewinkelte Ende 10, 11 des jeweiligen Klemmschenkels 8, 9 aufgeschoben bzw. aufgefädelt. Anschließend wird das Sicherungselement 20, 21 montiert. Daran anschließend wird der Lagerabschnitt 22, 23 auf den Klemmschenkel 8, 9 aufgeschoben, bis er gegen das Sicherungselement 20, 21 anliegt. Der Lagerabschnitt 22, 23 weist eine mittlere Bohrung auf, deren Durchmesser geringfügig größer ist als der Durchmesser eines Klemmschenkels 8, 9. Dadurch ist eine freie Drehbarkeit des Rollkörpers 18, 19 gewährleistet.

Der Lagerabschnitt 22, 23 weist bei der in den Fig. 5 bis 7 dargestellten Ausführungsform an einem Ende einen radialen Steg 26, 27 mit einem ringförmigen äußeren Teil 28, 29 auf, so daß ein Aufnahmebereich für das Verschlußteil 24, 25 gebildet wird. Der radiale Steg 26, 27 begrenzt eine kreisförmige Vertiefung 30, 31, die einen größeren Durchmesser aufweist als der Wellensicherungsring. Dies ermöglicht es, das Verschlußteil 24, 25 mit dem Lagerabschnitt 22, 23 an weniger Punkten zu verkleben und damit den Rollkörper 18, 19 gegen eine axiale Verschiebung an den Klemmschenkeln 8, 9 zu sichern. Voraussetzung für eine freie Drehbarkeit des Rollkörpers 18, 19 ist, daß dieser nicht mit dem Klemmschenkel 8, 9 verklebt wird.

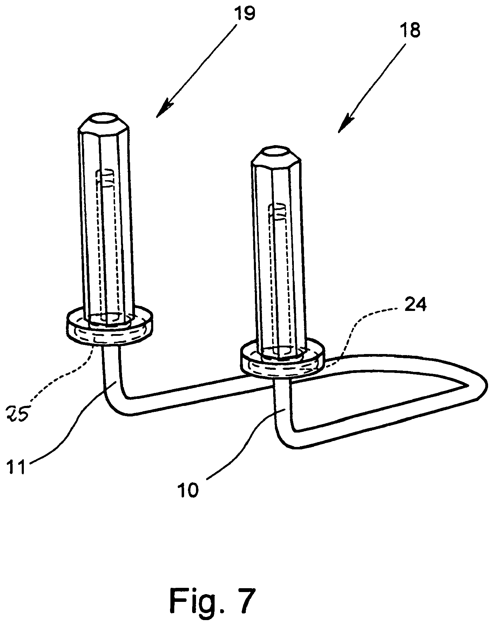

Um eine leichte Drehbarkeit der Rollkörper 18, 19 darüber hinaus zu gewährleisten, kann zwischen dem Lagerabschnitt 22, 23 und dem Verschlußteil 24, 25 nach dem Verkleben ein Hohlraum für das Sicherungselement 20, 21 gebildet sein, so daß eine geringfügige Ausgleichsbewegung des Rollkörpers 18, 19 in axialer Richtung relativ zu dem Sicherungselement 20, 21 möglich ist. In Fig. 5 sind die Rollkörper 18, 19 in demontiertem Zustand und in Fig. 7 im montierten Zustand dargestellt.

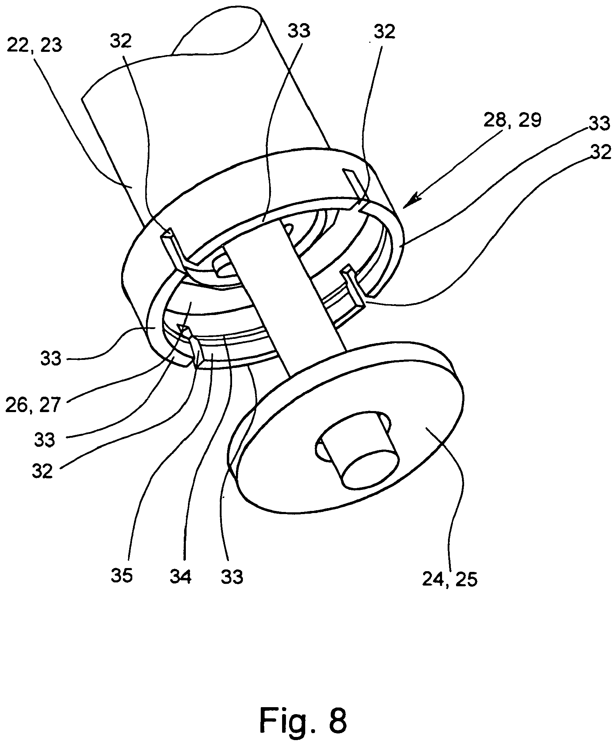

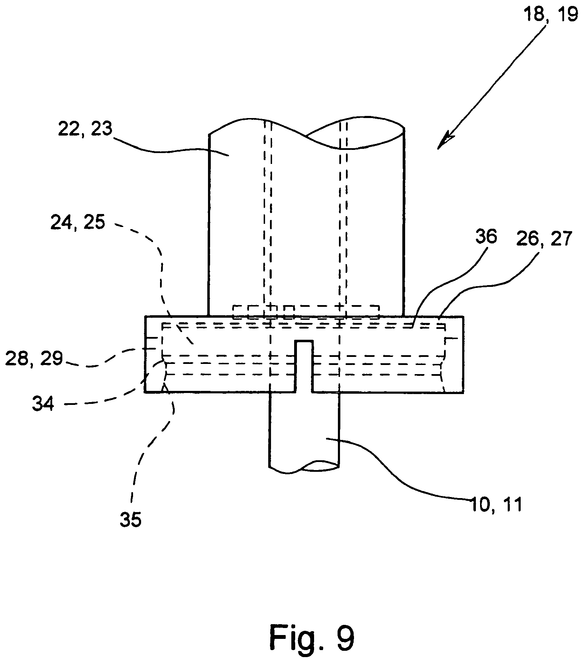

In den Fig. 8 und 9 ist eine alternative Ausführungsform eines Rollkörpers 18, 19 dargestellt. Entsprechend der oben beschriebenen Ausführungsform weist der Rollkörper 18, 19 wiederum einen Lagerabschnitt 22, 23 mit einem radialen Steg 26, 27 und mit einem ringförmigen äußeren Teil 28, 29 an seinem einem Verschlußteil 24, 25 zugewandten Ende auf. Im Unterschied zu der in den Fig. 5 bis 7 dargestellten Ausführungsform ist es bei der in den Fig. 8 und 9 dargestellten Ausführungsform jedoch vorgesehen, daß der Lagerabschnitt 22, 23 mit dem Verschlußteil 24, 25 durch eine Rastverbindung kraftschlüssig und formschlüssig verbunden wird. Zu diesem Zweck sind in dem ringförmigen äußeren Teil 28, 29 axiale Schlitze 32 vorgesehen, so daß der ringförmige äußere Teil 28, 29 in mehrere Kreissegmentabschnitte 33 unterteilt wird. Beim Eindrücken des Verschlußteils 24, 25 in den zwischen dem radialen Steg 26, 27 und dem ringförmigen äußeren Teil 28, 29 gebildeten Aufnahmebereich lassen sich die Kreissegmentabschnitte 33 gegen die Materialrückstellkräfte reversibel nach außen biegen, wobei die zu überwindenden Schnappkräfte durch die geschlitzte Ausführungsform gering sind.

Der ringförmige äußere Teil 28, 29 weist auf der Innenseite eine in axialer Richtung innenliegende Schräge 34 und eine außenliegende Schräge 35 auf. Dies ist insbesondere in Fig. 9 dargestellt. Die außenliegende Schräge 35 weist eine geringere Steigung auf als die innenliegende Schräge 34, was zum einen einen geringeren Kraftaufwand bei dem Eindrücken des Verschlußteils 24, 25 in den Aufnahmebereich und zum anderen eine ausreichende axiale Sicherung des Rollkörper 18, 19 nach dem Einrasten des Verschlußteils 24, 25 gewährleistet.

Auch bei der in den Fig. 8 und 9 dargestellten Ausführungsform ist ein Hohlraum 36 zwischen dem Verschlußteil 24, 25 und dem radialen Steg 26, 27 im montierten Zustand des Rollkörpers 18, 19 vorgesehen, um eine leichte Drehbarkeit des Rollkörpers 18, 19 zu gewährleisten. Die in den Fig. 8 und 9 dargestellte Ausführungsform ermöglicht eine einfache Montage der Rollkörper 18, 19. Im übrigen läßt sich das Verschlußteil 24, 25 für eine Demontage der Rollkörper 18, 19 bedarfsweise wieder von dem Lagerabschnitt 22, 23 trennen.

Der radiale Steg 26, 27 an dem Lagerabschnitt 22, 23 wirkt als Anschlag für die Klemmrolle 16, 17. Der Verschiebeweg der Klemmrolle 16, 17 auf dem Lagerabschnitt 22, 23 ist somit nach unten begrenzt.

Die in den Fig. 1 und 2 gezeigte Kinnstütze 6 kann in axialer Richtung des Atemrohres 2 ebenfalls tiefenverstellbar an dem Gehäuseteil 3 gelagert und an unterschiedlichen Stellen am Gehäuseteil 3 festsetzbar sein. Dadurch läßt sich die Position der Kinnstütze 6 an das Gesicht des Benutzers 5 anpassen und eine für den Benutzer 5 angenehme Stellung der Anwendereinheit 1 bei der Bestimmung der Leistungsparameter sicherstellen. Die Klemmung der Kinnstütze 6 am Gehäuseteil 3 kann der beschriebenen Klemmung der Nasenklemme 4 am Gehäuseteil 3 entsprechen.

Die Kinnstütze 6 kann einen im wesentlichen U-förmigen Stützbügel 37 mit zwei Stützschenkeln 38, 39 aufweisen, wobei die beiden Stützschenkel 38, 39 L-förmig gebogen und mit dem Gehäuseteil 3 verbunden sein können. Der Stützbügel 37 kann als geschlossene Drahtschleife ausgebildet sein.

Die beiden Stützschenkel 38, 39 sind unter Bildung eines im wesentlichen U-förmigen Abstützbereiches 40 zur Anlage gegen das Kinn des Benutzers 5 miteinander verbunden, wobei die beiden Stützschenkel 38, 39 im Abstützbereich 40 unter einem Winkel von kleiner oder gleich 90° zur Mittellängsachse X₁ des Atemrohrs 2 angeordnet sein können. Die Abbiegung der Stützschenkel 38, 39 im Abstützbereich 40 kann dabei der Abbiegung der freien Enden 10, 11 der Klemmschenkel 8, 9 der Nasenklemme 4 entsprechen. Dadurch wird ein angenehmer Tragezustand der Anwendereinheit 1 erreicht.

Wie sich weiter aus Fig. 2 ergibt, können die beiden Stützschenkel 38, 39 über eine Verschraubung mit wenigstens einer Klemmschraube an dem Gehäuseteil 3 festsetzbar sein. Hierbei können die Stützschenkel 38, 39 - entsprechend zur Klemmung der Nasenklemme 4 - zwischen einem Schraubenkopf 41 der Klemmschraube und dem Gehäuseteil 3 mit einem bestimmten Abstand zum Kinn des Benutzers 5 einspannbar sein.

Nicht dargestellt ist im einzelnen, daß an der Unterseite des Gehäuseteils 3 und/oder an dem Schraubenkopf 41 wenigstens ein Führungsvorsprung und/oder eine Führungsausnehmung zur Führung der Stützschenkel 38, 39 vorgesehen sein kann.

Nachfolgend sind im übrigen Merkmale und Aspekte der Erfindung angegeben, die für sich oder in beliebiger Kombination miteinander und/oder mit den in den Patentansprüchen angegebenen Merkmalen mögliche Ausführungsformen beschreiben und von daher auch erfindungswesentlich sind. So kann bei der erfindungsgemäßen Anwendereinheit vorgesehen sein,
- daß die freien Enden (10, 11) der Klemmschenkel (8, 9) unter einem Winkel von kleiner oder gleich 90° zur Mittellängsachse (X₁) des Atemrohrs (2) angeordnet sind und/oder
- daß der Klemmbügel (7) über eine Verschraubung mit wenigstens einer Klemmschraube an dem Gehäuseteil (3) festsetzbar ist, wobei die Klemmschraube zwischen den Klemmschenkeln (8, 9) in das Gehäuseteil (3) einschraubbar und der Klemmbügel (7) zwischen einem Schraubenkopf (15) der Klemmschraube und dem Gehäuseteil (3) einspannbar ist und/oder
- daß an dem Gehäuseteil (3) und/oder an dem Schraubenkopf (15) wenigstens ein Führungsvorsprung und/oder eine Führungsausnehmung zur Führung der Klemmschenkel (8, 9) vorgesehen ist und/oder
- daß die Klemmrolle (16, 17) zylinderförmig ist und/oder
- daß die Klemmrolle (16, 17) als elastischer Formkörper, insbesondere aus Schaumstoff, hergestellt ist und/oder
- daß die Klemmrolle (16, 17) in Richtung der Längsachse (X₂) des Klemmschenkels (8, 9) verschiebbar gelagert ist und/oder
- daß der Rollkörper (18, 19) eine größere Länge aufweist als die Klemmrolle (16, 17) und/oder
- daß wenigstens ein Sicherungselement (20, 21) zur Sicherung des Rollkörpers (18, 19) gegen axiales Verschieben auf dem Klemmschenkel (8, 9) vorgesehen ist, vorzugsweise ein in einer Nut des Klemmschenkels (8, 9) festgelegter Wellensicherungsring und/oder
- daß der Rollkörper (18, 19) zweiteilig ausgebildet ist und einen zylinderförmigen Lagerabschnitt (22, 23) und ein Verschlußteil (24, 25) aufweist, wobei der Lagerabschnitt (22, 23) drehbar an dem Klemmschenkel (8, 9) gelagert ist und gegen das Sicherungselement (20, 21) anliegt, wobei das Verschlußteil (24, 25) kraft- und/oder form- und/oder stoffschlüssig mit dem Lagerabschnitt (22, 23) verbunden ist und wobei, vorzugsweise, zwischen dem Lagerabschnitt (22, 23) und dem Verschlußteil (24, 25) ein Hohlraum für das Sicherungselement (20, 21) gebildet ist und/oder
- daß der Lagerabschnitt (22, 23) an seinem unteren Ende einen radialen Steg (26, 27) als Anschlag für die Klemmrolle (16, 17) aufweist und/oder
- daß die beiden Stützschenkel (38, 39) unter Bildung eines im wesentlichen U-förmigen Abstützbereiches (40) zur Anlage gegen das Kinn des Benutzers (5) miteinander verbunden sind, wobei der Abstützbereich (40) unter einem Winkel von kleiner oder gleich 90° zur Mittellängsachse (X₁) des Atemrohrs (2) angeordnet ist und/oder
- daß die beiden Stützschenkel (38, 39) über eine Verschraubung mit wenigstens einer Klemmschraube an dem Gehäuseteil (3) festsetzbar sind, wobei die Stützschenkel (38, 39) zwischen einem Schraubenkopf (41) der Klemmschraube und dem Gehäuseteil (3) einspannbar sind.

## Patentansprüche

1. Anwendereinheit (1) zur Bestimmung von Leistungsparametern aus Atemgasanalysen für den Einsatz in Spiroergometriegeräten, mit einem vorzugsweise auswechselbaren Atemrohr (2) und einem Gehäuseteil (3), wobei, weiter vorzugsweise, das Atemrohr (2) in das Gehäuseteil (3) einführbar ist, um die Anwendereinheit in einen Messzustand zu überführen, wobei auf der Oberseite des Gehäuseteils (3) eine mit dem Gehäuseteil (3) verbundene Nasenklemme (4) ausgebildet zum Verschließen der Nase eines Benutzers (5) bei der Bestimmung der Leistungsparameter und auf der Unterseite des Gehäuseteils (3) eine mit dem Gehäuseteil (3) verbundene Kinnstütze (6) ausgebildet zur Anlage gegen das Kinn eines Benutzers (5) bei der Bestimmung der Leistungsparameter vorgesehen sind, **dadurch gekenntzeichnet**, **dass** die Nasenklemme (4) in axialer Richtung des Atemrohres (2) tiefenverstellbar an dem Gehäuseteil (3) gelagert und an unterschiedlichen Stellen am Gehäuseteil (3) festsetzbar ist und dass die Kinnstütze (6) in axialer Richtung des Atemrohres (2) tiefenverstellbar an dem Gehäuseteil (3) gelagert und an unterschiedlichen Stellen am Gehäuseteil (3) festsetzbar ist.

2. Anwendereinheit nach Anspruch 1, **dadurch gekennzeichnet, dass** das Gehäuseteil (3) nach dem Einführen das Atemrohrs (2) in den Mund eines Probanden lediglich über die Nasenklemme (4) und das Atemrohr (2) am Kopf des Probanden haltbar und über die Kinnstütze (6) am Kinn des Probanden abstützbar ist.

3. Anwendereinheit nach Anspruch 1 oder 2, **dadurch gekennzeichnet, dass** die Nasenklemme (4) einen im wesentlichen U-förmigen Klemmbügel (7) mit zwei Klemmschenkeln (8, 9) aufweist, wobei die beiden Klemmschenkel (8, 9) an den freien Enden (10, 11) L-förmig abgewinkelt sind und wobei zwischen den abgewinkelten Enden (10, 11) ein zur Seite eines Mundstücks des Atemrohrs (2) offener Klemmbereich (12) zum Einführen der Nase gebildet wird.

4. Anwendereinheit nach einem der vorhergehenden Ansprüche, **dadurch gekennzeichnet, dass** jeder Klemmschenkel (8, 9) an seinem abgewinkelten Ende (10, 11) eine um die Längsachse (X₂) des Klemmschenkels (8, 9) drehbare Klemmrolle (16, 17) aufweist, wobei zwischen den Klemmrollen (16, 17) der Klemmbereich (12) gebildet wird.

5. Anwendereinheit nach einem der vorhergehenden Ansprüche, **dadurch gekennzeichnet, dass** an dem abgewinkelten Ende (10, 11) des Klemmschenkels (8, 9) ein um die Längsachse (X₂) des Klemmschenkels (8, 9) drehbar gelagerter zylindrischer Rollkörper (18, 19) für die Klemmrolle (16, 17) vorgesehen ist, wobei die Klemmrolle (16, 17) eine mittlere Öffnung zum Aufstecken auf den Rollkörper (18, 19) aufweist und, vorzugsweise, in axialer Richtung auf dem Rollkörper (18, 19) verschiebbar ist.

6. Anwendereinheit nach einem der vorhergehenden Ansprüche, **dadurch gekennzeichnet, dass** die Kinnstütze (6) einen im Wesentlichen U-förmigen Stützbügel (37) mit zwei Stützschenkeln (38, 39) aufweist, wobei die beiden Stützschenkel (38, 39) L-förmig gebogen und mit dem Gehäuseteil (3) verbunden sind.

7. Anwendereinheit nach einem der vorhergehenden Ansprüche 6 bis 8, **dadurch gekennzeichnet, dass** an dem Gehäuseteil (3) und/oder an dem Schraubenkopf (41) wenigstens ein Führungsvorsprung und/oder eine Führungsausnehmung zur Führung der Stützschenkel (38, 39) vorgesehen ist.

## Claims

1. User unit (1) for determining output parameters from respiratory gas analyses, for use in apparatus for ergospirometry, with a preferably replaceable breathing tube (2) and a housing part (3), wherein the breathing tube (2), again preferably, can be inserted into the housing part (3) in order to place the user unit in a measuring state, wherein a nose clip (4) connected to the housing part (3) and designed to seal the nose of a user (5) during the determination of the output parameters is provided on the top side of the housing part (3), and a chin rest (6) connected to the housing part (3) and designed to rest against the chin of a user (5) during the determination of the output parameters is provided on the bottom side of the housing part (3), **characterized in that** the nose clip (4) is mounted on the housing part (3) so as to be adjustable in depth in the axial direction of the breathing tube (2) and can be fixed at various positions on the housing part (3), and **in that** the chin rest (6) is mounted on the housing part (3) so as to be adjustable in depth in the axial direction of the breathing tube (2) and can be fixed at various points on the housing part (3).

2. User unit according to Claim 1, **characterized in that**, after the insertion of the breathing tube (2) into the mouth of a test subject, the housing part (3) can be held on the head of the test subject merely via the nose clip (4) and the breathing tube (2) and can be supported on the chin of the test subject via the chin rest (6).

3. User unit according to Claim 1 or 2, **characterized in that** the nose clip (4) has a substantially U-shaped clamping bracket (7) with two clamping arms (8, 9), wherein the two clamping arms (8, 9) are angled in an L shape at the free ends (10, 11), and wherein a clamping area (12) provided for insertion of the nose, and open towards a mouthpiece of the breathing tube (2), is formed between the angled ends (10, 11).

4. User unit according to one of the preceding claims, **characterized in that** each clamping arm (8, 9) has, at its angled end (10, 11), a clamp roller (16, 17) that can be rotated about the longitudinal axis (X₂) of the clamping arm (8, 9), wherein the clamping area (12) is formed between the clamp rollers (16, 17).

5. User unit according to one of the preceding claims, **characterized in that** a cylindrical roller body (18, 19) that is mounted so as to be rotatable about the longitudinal axis (X₂) of the clamping arm (8, 9) is provided, for the clamp roller (16, 17), on the angled end (10, 11) of the clamping arm (8, 9), wherein the clamp roller (16, 17) has a central opening for placement onto the roller body (18, 19) and is preferably movable on the roller body (18, 19) in the axial direction.

6. User unit according to one of the preceding claims, **characterized in that** the chin rest (6) has a substantially U-shaped support bracket (37) with two support arms (38, 39), wherein the two support arms (38, 39) are bent in an L shape and connected to the housing part (3).

7. User unit according to one of the preceding claims 6 to 8, **characterized in that** at least one guide projection and/or guide recess, for guiding the support arms (38, 39), is provided on the housing part (3) and/or on the screw head (41).

## Revendications

1. Unité d'utilisateur (1) pour la détermination de paramètres de performance dans des analyses des gaz de respiration destinée à être à utilisée dans des appareils de spiroergométrie, avec un tube respiratoire de préférence remplaçable (2) et une partie de boîtier (3), dans laquelle, de préférence encore, le tube respiratoire (2) peut être introduit dans la partie de boîtier (3), afin de placer l'unité d'utilisateur dans un état de mesure, dans laquelle il est prévu sur le côté supérieur de la partie de boîtier (3) une pince nasale (4) reliée à la partie de boîtier (3) configurée en vue de fermer le nez d'un utilisateur (5) lors de la détermination des paramètres de performance et sur le côté inférieur de la partie de boîtier (3) un appui de menton (6) relié à la partie de boîtier (3) configuré pour s'appliquer contre le menton d'un utilisateur (5) lors de la détermination des paramètres de performance, **caractérisée en ce que** la pince nasale (4) est montée sur la partie de boîtier (3) de façon réglable en profondeur dans la direction axiale du tube respiratoire (2) et peut être calée en différents endroits sur la partie de boîtier (3) et **en ce que** l'appui de menton (6) est monté sur la partie de boîtier (3) de façon réglable en profondeur dans la direction axiale du tube respiratoire (2) et peut être calé en différents endroits sur la partie de boîtier (3).

2. Unité d'utilisateur selon la revendication 1, **caractérisée en ce que**, après l'introduction du tube respiratoire (2) dans la bouche d'un probant, la partie de boîtier (3) ne peut être tenue sur la tête du probant que par la pince nasale (4) et le tube respiratoire (2) et appuyée contre le menton du probant par l'appui de menton (6).

3. Unité d'utilisateur selon la revendication 1 ou 2, **caractérisée en ce que** la pince nasale (4) présente un étrier de pinçage (7) essentiellement en forme de U avec deux branches de pinçage (8, 9), dans laquelle les deux branches de pinçage (8, 9) sont coudées en forme de L aux extrémités libres (10, 11) et dans laquelle une zone de pinçage (12) ouverte vers le côté d'un embout buccal du tube respiratoire (2) est formée entre les extrémités coudées (10, 11) pour y insérer le nez.

4. Unité d'utilisateur selon l'une quelconque des revendications précédentes, **caractérisée en ce que** chaque branche de pinçage (8, 9) présente à son extrémité coudée (10, 11) un rouleau de pinçage (16, 17) pouvant tourner autour de l'axe longitudinal (X₂) de la branche de pinçage (8, 9), dans laquelle la zone de pinçage (12) est formée entre les rouleaux de pinçage (16, 17).

5. Unité d'utilisateur selon l'une quelconque des revendications précédentes, **caractérisée en ce qu'**il est prévu sur l'extrémité coudée (10, 11) de la branche de pinçage (8, 9) un corps de roulement cylindrique (18, 19) pour le rouleau de pinçage (16, 17), monté de façon rotative autour de l'axe longitudinal (X₂) de la branche de pinçage (8, 9), dans laquelle le rouleau de pinçage (16, 17) présente une ouverture centrale destinée à l'engagement sur le corps de roulement (18, 19), et peut se déplacer de préférence en direction axiale sur le corps de roulement (18, 19).

6. Unité d'utilisateur selon l'une quelconque des revendications précédentes, **caractérisée en ce que** l'appui de menton (6) présente un étrier d'appui essentiellement en forme de U (37) avec deux branches d'appui (38, 39), dans laquelle les deux branches d'appui (38, 39) sont courbées en forme de L et sont reliées à la partie de boîtier (3).

7. Unité d'utilisateur selon l'une quelconque des revendications précédentes 6 à 8, **caractérisée en ce qu'**il est prévu sur la partie de boîtier (3) et/ou sur la tête de vis (41) au moins une saillie de guidage et/ou un évidement de guidage pour le guidage des branches d'appui (38, 39).
